# DEMANDE DE BREVET EUROPEEN

(11) **EP 4 725 472 A1**
(43) Date de publication de la demande: **15.04.2026**
(21) Numéro de dépôt: 25208693.9
(22) Date de dépôt: 14.10.2025
(51) Int. Cl.: A61K 8/04, A61K 8/34, A61K 8/55, A61K 8/73, A61Q 15/00, A61Q 19/10

(54) **COMPOSITION COSMÉTIQUE POUR SOINS CORPORELS SANS RINÇAGE**

(30) Priorité: 14.10.2024 FR 2411096
(71) Demandeur: Nowa Cosmetics, 33300 Bordeaux (FR)
(72) Inventeur: WYDRA, Jessica, 33480 SAINTE HELENE (FR)
(74) Mandataire: Aquinov

(57) **Abrégé**

La présente invention concerne une composition cosmétique pour soins corporels se présentant sous la forme d'une solution nettoyante gélifiée, sans rinçage, celle-ci comprenant au moins 90% d'eau, notamment une eau d'origine végétale, telle qu'une eau de fruit, une eau florale etc., entre 1,5% et 4% d'au moins un conservateur et entre 0,35% et 0,45% d'au moins un agent gélifiant ; et plus particulièrement une composition cosmétique, sans rinçage, pour la toilette et l'entretien du corps et des cheveux.

## Description

### Domaine technique

L'invention concerne une composition cosmétique pour soins corporels se présentant sous la forme d'une solution nettoyante gélifiée, celle-ci comprenant au moins 90% d'eau, en particulier une eau d'origine végétale, telle qu'une eau de fruit, une eau florale, ou encore une eau de légumes, entre 1,5% et 4% d'au moins un conservateur et entre 0,35% et 0,45% d'au moins un agent gélifiant. Plus particulièrement l'invention vise une composition cosmétique, sans rinçage, pour la toilette et l'hygiène corporelle.

### Etat de l'art

Les produits cosmétiques d'hygiène corporelle sans rinçage, tels que les shampoings secs ou encore les après-shampoings sans rinçage, ont gagné en popularité ces dernières années en raison de leur praticité et de leur impact environnemental plus limité, notamment sur leur consommation en eau. A titre d'exemple, environ 70% de l'empreinte carbone d'un shampoing provient de l'énergie nécessaire pour chauffer l'eau utilisée durant une douche.

Longtemps cantonné à des usages très spécifiques, notamment dans le domaine hospitalier, afin de proposer des soins d'hygiène aux personnes hospitalisées, par exemple alitées ou à mobilité réduite, ces produits répondent désormais à un besoin croissant de solutions d'hygiène rapides et faciles à utiliser, particulièrement adaptées aux modes de vie actifs et aux situations où l'accès à l'eau est limité, voire inexistant.

Traditionnellement, les produits cosmétiques d'hygiène corporelle nécessitent de l'eau pour être rincés après application, y compris pour les produits de type solide ou à reconstituer dans l'eau. Cependant, l'industrie cosmétique a développé des formulations innovantes permettant un nettoyage corporel efficace sans nécessité de rinçage. Ces formulations comprennent généralement des agents nettoyants doux, des hydratants, et des ingrédients qui s'évaporent rapidement pour laisser la peau propre et fraîche. Certaines formulations peuvent également inclure des agents antimicrobiens pour assurer une hygiène optimale. Toutefois, la majorité des produits sans rinçage connue se présente sous la forme de solution nettoyante très liquide, à appliquer soit en spray, soit sur des gants de toilettes, notamment dans le domaine hospitalier.

Les innovations récentes dans le domaine des produits sans rinçage se sont essentiellement concentrées sur le secteur du capillaire (soins cheveux et après-shampoings), répondant ainsi aux attentes des consommateurs pour des produits plus respectueux de l'environnement et limitant la consommation en eau.

Or, un défi majeur pour faciliter la généralisation de ces produits est de trouver un équilibre entre l'efficacité du nettoyage et la douceur pour la peau, tout en évitant la sensation de résidus après utilisation. La stabilité de la formulation et sa capacité à maintenir ses propriétés et son intégrité dans le temps sont également des aspects cruciaux.

Dans ce contexte et malgré les dernières avancées dans le domaine, les consommateurs restent à la recherche de produits d'hygiène corporelle améliorés comprenant des ingrédients naturels, offrant un nettoyage efficace, tout en réduisant l'empreinte hydrique. A ce jour, peu de solutions existent pour se laver en l'absence d'un accès à l'eau ou pour diminuer sa consommation d'eau quotidienne.

Les produits cosmétiques sous forme solide ou sous forme sèche à reconstituer répondent qu'en partie à cette problématique. En effet, ils nécessitent d'être rincés ou d'être reconstitués dans l'eau, impliquant la nécessité d'avoir accès à un point d'eau. Ces solutions ne répondent donc pas entièrement au besoin de pouvoir se laver en tout lieu et à tout instant de la journée.

Il existe donc un besoin pour des produits d'hygiène corporelle sans rinçage, contribuant alors à la préservation des ressources en eau, ce qui devient primordial en France, en Europe et dans le reste du monde afin de répondre aux nombreux défis environnementaux majeurs de notre époque. En effet, la demande en eau potable dépassera l'offre de 40% dès 2030, selon le dernier rapport des Nations Unies.

Pour répondre à ce besoin, la présente invention, dans une démarche de respect de l'environnement et de la préservation des ressources, a pour objet une composition cosmétique destinée aux soins corporels et plus particulièrement des produits cosmétiques de type gel douche, shampoing, ou après-shampoing pour le nettoyage et l'entretien du corps incluant le cuir chevelu et les cheveux.

### Résumé de l'invention

Ainsi, l'invention concerne une composition cosmétique, sous forme d'une solution nettoyante gélifiée comprenant :
- au moins 90% d'eau, en poids du poids total de la composition,
- au moins un conservateur, et
- au moins un agent gélifiant.

Ladite composition présente préférentiellement une viscosité comprise entre 40 et 95 mPa.s, plus préférentiellement entre 55 et 80 mPa.s.

Selon un autre objet, l'invention concerne une composition cosmétique, sous forme d'une solution nettoyante gélifiée comprenant :
- au moins 90% d'eau, en poids du poids total de la composition,
- entre 1,5% et 4% d'au moins un conservateur, en poids du poids total de la composition, et
- entre 0,35% et 0,45% d'au moins un agent gélifiant, en poids du poids total de la composition.

La présente composition selon l'invention est particulièrement destinée au soin corporel, incluant le soin capillaire, pour une toilette complète du visage, du corps et des cheveux. Selon un objet préféré, la composition selon l'invention est de type sans rinçage.

De façon préféré, l'eau est choisie parmi le groupe constitué par l'eau déminéralisée, l'eau déionisée, l'eau purifiée, l'eau distillée, l'eau thermale, l'eau minérale et l'eau d'origine végétale. Plus préférentiellement, l'eau d'origine végétale est choisie parmi le groupe constitué de l'eau de fruit, l'eau de légume, l'eau florale, l'eau végétale, l'eau alimentaire et leurs mélange.

L'invention se caractérise très préférentiellement par l'utilisation d'une eau upcyclée, notamment une eau d'origine végétale comme base principale, remplaçant avantageusement l'eau ajoutée (eau potable déminéralisée) traditionnellement présente dans les produits cosmétiques, y compris dans les solutions nettoyantes sans rinçage connues, notamment dans le domaine hospitalier. Cette approche permet d'obtenir une composition concentrée en ingrédients tout en maintenant une texture adaptée à une application sans rinçage et renforçant encore plus la réduction de l'empreinte hydrique du produit. Par ailleurs, il n'existe pas de produits d'hygiène corporelle sous forme liquide, sans eau potable (eau ajoutée) dans sa formulation et ne nécessitant pas de rinçage.

Ainsi, selon un objet particulièrement préféré, l'invention concerne une composition cosmétique, sous forme d'une solution nettoyante gélifiée sans eau ajoutée, comprenant :
- au moins 90% d'eau d'origine végétale choisie parmi le groupe constitué d'une eau de fruit, d'une eau de légume, d'une eau florale, d'une eau végétale, d'une eau alimentaire et leurs mélange, en poids du poids total de la composition,
- entre 1,5% et 4% d'au moins un conservateur, en poids du poids total de la composition, et
- entre 0,35% et 0,45% d'au moins un agent gélifiant, en poids du poids total de la composition.

Selon un autre objet préféré, la composition selon l'invention comprend un mélange spécifique d'au moins trois conservateurs, très préférentiellement le sodium benzoate, le sodium phytate et le pentylène glycol, permettant de préserver l'intégrité du produit, y compris lors de l'absence d'eau ajoutée, tel que l'eau déminéralisée ou l'eau purifiée. En effet, l'eau naturelle d'origine végétale ne présente pas le même degré de pureté que l'eau déminéralisée, l'eau purifiée ou potable, ce qui entraîne alors un risque accru de prolifération microbienne. L'inventeur a ainsi spécifiquement sélectionné un mélange de trois conservateurs pour prévenir toute dégradation et maintenir l'intégrité du produit, y compris lorsqu'il comprend de l'eau d'origine végétale. Enfin, ce mélange permet également de réduire le risque d'oxydation.

Ainsi, selon un objet particulièrement préféré de l'invention, les conservateurs compris dans la composition selon l'invention sont :
- le pentylène glycol entre 1,5% et 2,5%, en poids du poids total de la composition,
- le sodium benzoate entre 0,2% et 0,4%, en poids du poids total de la composition, et
- le sodium phytate à hauteur d'au plus 0,25%, en poids du poids total de la composition.

Selon un autre objet, la présente composition comprend également au moins un agent gélifiant. Préférentiellement l'agent gélifiant est la gomme de xanthane à hauteur de 0,35% à 0,45%, en poids du poids total de la composition. Plus préférentiellement on choisira la gomme de xanthane RHODICARE^{®} D (OUVRICARE D), à savoir une gomme de xanthane obtenu à partir de *Xanthomonas campestris,* notamment la gomme de xanthane CAS N°11138-66-2, qui confère à la composition selon l'invention sa texture caractéristique laissant notamment un rendu frais, agréable et non collant sur la peau.

De façon particulièrement préféré, la composition comprend entre 0,35% et 0,45% de gomme de xanthane RHODICARE^{®} D (OUVRICARE D), encore plus spécifiquement 0,4% (plus ou moins 0,02%) de gomme de xanthane RHODICARE^{®} D (OUVRICARE D), en poids du poids total de la composition.

Selon un autre objet préféré de l'invention, l'eau d'origine végétale apportant ses propriétés bénéfiques à la formulation, outre ses propriétés aqueuses, est avantageusement :
- une eau de fruit, telle qu'une eau de pomme, une eau de raisin, une eau de prune, et/ou
- une eau de légume telle qu'une eau de concombre, et/ou
- une eau de fleur telle que l'eau de rose, eau de lavande ou eau de fleur d'oranger, et/ou
- une eau végétale telle qu'une eau d'aloé vera, une eau de coco, une eau de thé vert, une eau de bambou, et/ou
- une eau alimentaire telle que l'eau de riz, l'eau d'avoine.

Pour améliorer davantage les propriétés cosmétiques, l'expérience utilisateur, ou encore la stabilité dans le temps, la composition selon l'invention peut également comprendre au moins un agent antioxydant, et/ou au moins un agent solubilisant, et/ou au moins un parfum.

La composition cosmétique selon la présente invention répond ainsi aux différentes attentes des consommateurs tout en surmontant les défis imposés par les produits cosmétiques sans rinçage, notamment concernant leurs formulations. En outre, celle-ci permet également de surmonter les difficultés rencontrées par les formulations dépourvues d'eau ajoutée, notamment en termes de stabilité, ou de texture. Ainsi, la composition cosmétique selon la présente invention maintient une efficacité de nettoyage optimale, présente une texture agréable et facile à appliquer, reste stable dans le temps et utilise des ingrédients naturels et durables, qu'elle comprenne une eau issue du surcyclage ou non. La présente composition répond ainsi aux exigences modernes en matière de soin corporel, d'efficacité et de durabilité.

Ainsi, selon un autre aspect, l'invention trouve son application dans le domaine des produits cosmétiques d'hygiène corporelle, avantageusement pour l'élimination des mauvaises odeurs, du sébum et de la transpiration.

Ces utilisations cosmétiques polyvalentes répondent ainsi aux besoins variés des consommateurs en matière d'hygiène corporelle rapide, en particulier des consommateurs ayant des besoins en matière de soin corporel quotidien tout en n'ayant pas accès à un point d'eau permettant une hygiène corporelle traditionnelle.

L'invention vise donc une utilisation cosmétique de la composition selon l'invention, pour éliminer les mauvaises odeurs, et/ou le sébum, et/ou la transpiration.

D'autres caractéristiques et avantages ressortiront de la description détaillée de l'invention, des exemples et des figures qui vont suivre.

### Description détaillée de l'invention

### Définition

Par « eau ajoutée » au sens de l'invention, on entend l'eau déminéralisée, l'eau potable, ou toute autre forme d'eau purifiée ou traitée qui est intentionnellement incorporée à une composition cosmétique en tant que base ou support d'une composition cosmétique traditionnelle sous forme aqueuse ou gélifiée. Ce terme exclut ainsi l'eau naturellement présente dans les ingrédients d'origine végétale tels que les eaux de fruits, les eaux de légumes, les eaux florales, ou les eaux végétales, ou encore les eaux alimentaires ainsi que l'eau liée ou l'eau de constitution qui fait partie intégrante de la structure moléculaire de certains ingrédients.

Par « sans rinçage » au sens de l'invention, on entend une propriété de la composition cosmétique selon laquelle le produit est conçu pour être appliqué sur la peau ou les cheveux et laissé en place sans nécessiter d'élimination par rinçage à l'eau. Les produits sans rinçage sont notamment formulés pour être absorbés rapidement par la peau ou les cheveux, ne laissant pas de résidu visible ou collant, et sont particulièrement adaptés pour une utilisation pratique et rapide dans la routine d'hygiène corporelle quotidienne.

Par « gomme de xanthane RHODICARE^{®} D » ou « gomme de xanthane OUVRICARE D » au sens de l'invention, on entend une gomme de xanthane spécifiquement obtenu à partir de *Xanthomonas campestris,* notamment commercialisé par la société PMC OUVRIE. Très préférentiellement ladite gomme de xanthane est la gomme de xanthane ayant le CAS N°11138-66-2.

Par « soins corporels » au sens de l'invention, on entend l'ensemble des applications cosmétiques destinées au nettoyage, à l'hygiène et à l'entretien de la peau, des cheveux, des ongles ou des poils du corps humain, incluant le visage, le tronc, les membres, le cuir chevelu et les cheveux.

### Composition selon l'invention

La présente invention a donc pour objet une composition sous forme d'une solution nettoyante gélifiée comprenant au moins 90% d'eau, en poids du poids total de la composition, au moins un conservateur et au moins un agent gélifiant, ladite composition présentant avantageusement une viscosité comprise entre 40 et 95 mPa.s, très avantageusement entre 55 et 80 mPa.s.

Selon un objet particulier, la présente invention concerne une composition sous forme d'une solution nettoyante gélifiée comprenant :
- au moins 90% d'eau, en poids du poids total de la composition,
- entre 1,5% et 4% d'au moins un conservateur, en poids du poids total de la composition,
- entre 0,35% et 0,45% d'au moins un agent gélifiant, en poids du poids total de la composition.

Préférentiellement, la composition est de type sans rinçage.

Traditionnellement, les compositions cosmétiques, y compris les gels douche sans rinçage, contiennent généralement une quantité significative d'eau déminéralisée ou d'eau potable comme base. Cette eau ajoutée sert de support aux autres ingrédients et contribue à la texture et à l'application du produit. Cependant, l'utilisation d'eau ajoutée augmente l'empreinte environnementale du produit, notamment en termes de fabrication et de transport. A titre d'exemple, la France vend plus de 2 milliards de produits cosmétiques par an, dont l'eau potable est l'ingrédient principal. Ainsi, selon un mode de réalisation préféré de l'invention, lorsque l'on souhaite réduire au minimum l'empreinte hydrique, la composition comprend de l'eau upcyclée, notamment de l'eau d'origine végétale. La présente composition est alors dépourvue d'eau ajoutée, réduisant au strict minimum l'empreinte environnementale du produit.

Ainsi, la composition se présente préférentiellement sous la forme d'une solution nettoyante gélifiée, sans eau ajoutée, comprenant :
- au moins 90% d'eau upcyclée, en poids du poids total de la composition,
- entre 1,5% et 4% d'au moins un conservateur, en poids du poids total de la composition,
- entre 0,35% et 0,45% d'au moins un agent gélifiant, en poids du poids total de la composition.

De façon particulièrement préféré, l'eau upcyclée est une eau d'origine végétale. L'eau d'origine végétale intégrée dans la composition selon l'invention est ainsi préférentiellement choisie parmi le groupe constitué d'une eau de fruit, d'une eau de légume, d'une eau florale, d'une eau végétale, d'une eau alimentaire et leur mélange. Il en résulte que l'eau d'origine végétale n'est pas une eau ajoutée de type eau déminéralisée ou eau purifié etc. Par ailleurs, cette eau d'origine végétale représente au moins 90% du poids total de la composition, préférentiellement au moins 95% en poids du poids total de la composition, ce qui permet d'obtenir une formulation hautement concentrée en ingrédients naturels, réduisant l'empreinte hydrique et diminuant drastiquement l'utilisation des ressources naturelles en eau, répondant ainsi aux attentes des consommateurs.

L'utilisation d'eau d'origine végétale comme base principale de la composition permet ainsi de s'affranchir du besoin en eau ajoutée dans la présente composition cosmétique, réduisant alors minimum son empreinte environnementale. En effet, l'eau d'origine végétale utilisée dans la composition selon l'invention est une eau issue d'une démarche de surcyclage, (en anglais « upcycling »), c'est à dire que l'eau utilisée provenant de fruit aurait été jetée sans cette intégration dans la formulation cosmétique. Cette utilisation permet ainsi d'avoir une solution aqueuse remplissant les fonctionnalités de l'eau potable, sans venir puiser davantage dans les ressources naturelles en eau.

Préférentiellement, l'eau d'origine végétale est choisi parmi le groupe constitué de l'eau de pomme (*Malus domestica*), l'eau de raisin (*Vitis vinifera*), l'eau de coco (*Cocos nucifera*), l'eau de pastèque (*Citrullus lanatus*), l'eau de concombre (*Cucumis sativus*)*,* l'eau de rose (*Rosa damascena*), l'eau de lavande (*Lavandula angustifolia*), l'eau de fleur d'oranger (*Citrus aurantium*)*,* l'eau de camomille (*Matricaria chamomilla*), l'eau d'aloe vera, l'eau de bambou (*Bambusa vulgaris*)*,* l'eau de bouleau (*Betula alba*), l'eau de riz, l'eau d'avoine et leur mélange.

Dans un mode de réalisation particulièrement préféré, l'eau d'origine végétale est une eau de fruit, plus préférentiellement l'eau de fruit utilisée est une eau de pomme ou une eau de raisin. L'eau de pomme ou l'eau de raisin apporte avantageusement une base agréable pour la composition selon la présente invention.

Toutefois, la présence d'eau d'origine végétale, tel que de l'eau de fruit peut entraîner des effets indésirables plus important qu'avec de l'eau ajoutée dans le contexte de produits cosmétiques, notamment de produits destinés aux soins corporels. En effet, celle-ci peut entrainer un risque accru de contamination bactérienne, une diminution de la stabilité du produit. Il est également nécessaire d'éviter toute sensation désagréable sur la peau, que ce soit l'apparition de rougeurs voire de démangeaisons, ou encore la présence de résidus sur la peau.

Ainsi, la présente composition cosmétique selon l'invention comprend également au moins un conservateur et au moins un gélifiant, présentes dans des quantités spécifiques permettant de limiter le risque de contamination bactérienne, y compris lorsque l'eau est de l'eau d'origine végétale et ainsi surmonter les inconvénients liés au remplacement de l'eau d'origine végétale à la place de l'eau ajoutée. Ladite composition cosmétique selon l'invention limite ainsi au maximum le risque de contamination bactérienne que ladite composition comprenne de l'eau ajoutée ou de l'eau d'origine végétale.

Ainsi, la composition comprend avantageusement entre 1,5% et 4% d'au moins un conservateur, en poids du poids total de la composition, et entre 0,35% et 0,45% d'au moins un agent gélifiant, en poids du poids total de la composition.

Dans un mode de réalisation particulièrement préféré, la composition comprend un mélange d'au moins trois conservateurs spécifiquement sélectionné : le sodium benzoate, le sodium phytate et le pentylène glycol. Ce mélange spécifique permet de préserver efficacement l'intégrité du produit notamment lors de l'absence d'eau ajoutée, tout en minimisant les risques d'irritation cutanée.

Cet effet est encore amélioré lorsque les proportions de ces conservateurs sont comprises :
- Entre 1,5% et 2,5% de pentylène glycol, préférentiellement entre 1,8% et 2,2%, encore plus préférentiellement 2%, en poids du poids total de la composition,
- Entre 0,2% et 0,4% de sodium benzoate, préférentiellement entre 0,25% et 0,35%, encore plus préférentiellement 0,3%, en poids du poids total de la composition,
- Au plus 0,25% de sodium phytate, préférentiellement au plus 0,15%, encore plus préférentiellement au plus 0,1%, en poids du poids total de la composition.

La composition selon l'invention comprend également entre 0,35% et 0,45% en poids d'au moins un agent gélifiant. Dans un mode de réalisation particulièrement préféré, l'agent gélifiant utilisé est la gomme de xanthane, plus spécifiquement la gomme de xanthane RHODICARE^{®} D (OUVRICARE D).

La sélection de la gomme de xanthane, notamment la gomme de xanthane RHODICARE^{®} D (OUVRICARE D), à hauteur de 0,35-0,45%, plus préférentiellement 0,4% confère à la composition sa texture gélifiée caractéristique, permettant une application facile et une sensation agréable sur la peau. Par sensation agréable, on entend notamment le fait que la composition selon l'invention ne laisse aucun résidu indésirable après application, propriété fondamentale pour un produit cosmétique de soins corporels sans rinçage. En outre, la texture gélifiée facilite l'application sans rinçage du produit. A l'inverse une forme trop liquide entraîne une perte importante de produits lors de l'application et s'apparente plus à un produit de type gel hydroalcoolique pour les mains non adapté pour le lavage du corps, et une forme trop épaisse est désagréable à l'application, laissant une sensation collante, voire de nombreux résidus sur la peau.

Ainsi, la composition permet avantageusement une facilité d'application, un étalement sans effort excessif, une absence de résidus sur la peau après l'application.

Ainsi, la composition présente préférentiellement une viscosité comprise entre 20 et 1 000 mPa.s, préférentiellement entre 20 et 500 mPa.s, plus préférentiellement entre 30 et 100 mPa.s, encore plus préférentiellement entre 40 et 95 mPa.s, de manière particulièrement préféré entre 55 et 80 mPa.s.

Selon un mode de réalisation particulièrement préféré, la composition comprend 0,4% de gomme de xanthane RHODICARE^{®} D (OUVRICARE D), en poids du poids total de la composition.

La composition selon l'invention présente ainsi une texture « gel » très différente des solutions sans rinçage existante sur le marché, souvent très liquide. Ladite composition présente une texture gel agréable laissant sur la peau une sensation non grasse et non collante particulièrement recherchée et délicate à obtenir pour un produit de soins corporels sans rinçage.

Très préférentiellement, ladite composition selon l'invention est de type sans rinçage. Le produit est donc conçu pour être appliqué sur la peau et laissé en place sans nécessiter d'élimination par rinçage à l'eau ou par essuyage, permettant ainsi de répondre aux attentes des consommateurs à la recherche de solutions pour se laver n'importe où, et ainsi de surmonter les inconvénients précités.

Selon un autre objet, la composition peut comprendre des additifs optionnels pour améliorer ses propriétés et l'expérience utilisateur :
- au moins un agent antioxydant, et/ou
- au moins un agent solubilisant, et/ou
- au moins un parfum.

L'ajout d'un agent antioxydant permet de renforcer la stabilité de la formulation et d'offrir une protection supplémentaire contre les phénomènes d'oxydation. L'ajout d'un parfum permet d'améliorer l'expérience sensorielle du produit, le rendant plus agréable à utiliser.

La composition se présente avantageusement sous la forme d'un gel douche, d'un shampoing, d'un après-shampoing, d'un produit intime, ou encore d'un gel nettoyant visage.

Selon un autre aspect, la composition selon l'invention peut être obtenu par tout procédé connu de l'homme du métier. En particulier, le procédé de préparation de la composition selon l'invention peut comprendre les étapes suivantes :
a. Préparation de la phase aqueuse :
   i. Dans un récipient propre, verser la quantité requise d'eau, notamment d'eau d'origine végétale (eau de fruit, de légume, florale ou végétale), représentant au moins 90% du poids total de la composition,
   ii. Optionnellement, chauffer l'eau à une température comprise entre 40°C et 50°C sous agitation constante,
b. Ajout de l'agent gélifiant :
   i. Dans le récipient, préparer une dispersion de la gomme de xanthane dans l'eau d'origine végétale,
   ii. Incorporer lentement la totalité de la gomme de xanthane sous agitation pour éviter la formation de grumeaux,
   iii. Poursuivre l'agitation jusqu'à obtention d'une texture homogène et gélifiée,
c. Incorporation des conservateurs :
   i. Ajouter progressivement le pentylène glycol (entre 1,5% et 2,5% du poids total) à la phase aqueuse, en maintenant l'agitation,
   ii. Incorporer ensuite le sodium benzoate (entre 0,2% et 0,4% du poids total) en s'assurant de sa complète dissolution,
   iii. Ajouter enfin le sodium phytate (au plus 0,25% du poids total) en poursuivant l'agitation jusqu'à dissolution complète,
d. Eventuellement, incorporation des additifs optionnels :
   i. Ajouter l'agent solubilisant choisi en respectant les proportions recommandées,
   ii. Incorporer le parfum, le cas échéant, en veillant à une répartition homogène dans la composition,
e. Homogénéisation finale :
   i. Maintenir l'agitation pendant 15 à 20 minutes supplémentaires pour assurer une parfaite homogénéité de la composition,
f. Contrôle qualité :
   i. Vérifier le pH de la composition et l'ajuster si nécessaire pour qu'il soit compatible avec une application cutanée,
   ii. Contrôler la viscosité et la texture de la composition pour s'assurer qu'elle correspond aux spécifications désirées.

Ce procédé permet d'obtenir la composition selon l'invention gélifiée homogène, stable et prête à l'emploi. Il est important de noter que toutes les étapes doivent être réalisées dans des conditions d'hygiène strictes pour garantir la qualité microbiologique du produit final.

Selon un autre aspect, de nombreuses utilisations de la composition selon l'invention sans rinçage sont possibles, offrant une solution pratique pour l'hygiène personnelle dans diverses situations.

Ainsi, la composition selon l'invention est avantageusement utilisée :
- pour une hygiène quotidienne rapide, par exemple pour un rafraîchissement rapide entre deux activités, comme alternative à une douche quotidienne,
- lors d'activités sportives, par exemple pour une toilette après une séance d'entraînement, pour les athlètes en déplacement n'ayant pas accès à des installations de douche
- lors de voyages, par exemple pour les longs trajets en avion, train ou voiture, ou encore dans des situations de camping ou de randonnée où l'accès à l'eau est limité
- pour toutes les activités de plein air dans lesquelles l'accès à l'eau est compliqué (camping, van, camping-car, bivouac)
- en milieu hospitalier, par exemple pour l'hygiène des patients alités ou à mobilité réduite, ou encore comme solution de nettoyage rapide et alternative pour le personnel soignant
- lors de situations d'urgence, par exemple lors de coupures d'eau ou de restrictions d'utilisation d'eau, dans des zones sinistrées où l'accès à l'eau courante est temporairement impossible
- lors d'événements en plein air, par exemple lors de festivals de musique, concerts, événements sportifs où les installations sanitaires sont limitées
- en cas de handicap ponctuel, comme un bras ou une jambe cassée, complexifiant la tenue d'une bonne hygiène corporelle
- pour les soins des personnes âgées ou dépendantes en facilitant l'hygiène quotidienne des personnes à mobilité réduite, limitant ainsi le déplacement vers une salle de bain.
- pour la toilette intime, en remplacement des lingettes intimes, celles-ci étant une catastrophe environnementale
- pour le nettoyage ponctuel des enfants à partir de 3 ans
- pour l'industrie du tourisme confrontée au besoin de limiter la consommation d'eau, comme les campings, mais également les gîtes, refuges, auberges de jeunesses...

Ces nombreuses utilisations démontrent la polyvalence et l'intérêt d'un tel produit de soins corporels, répondant ainsi de manière générale à un besoin d'hygiène personnelle dans un contexte où l'accès à l'eau ou le temps pour y accéder est limité.

Selon un autre objet la composition selon l'invention présente plusieurs effets bénéfiques, à savoir :
- L'élimination des mauvaises odeurs : La formulation aide à neutraliser les odeurs corporelles indésirables, et/ou
- L'élimination ou la réduction des impuretés : sébum, transpiration, toutes les sécrétions du corps. La composition aide ainsi à réguler la production de sébum, contribuant à une peau plus nette.

Selon un autre aspect, l'invention vise une méthode de nettoyage corporel sans consommation d'eau supplémentaire comprenant l'application de la composition gélifiée selon l'invention. Préférentiellement, la méthode comprend une étape d'élimination sans rinçage et/ou sans essuyage.

L'invention est à présent illustrée par des exemples non limitatifs de compositions selon l'invention et par des résultats.

### Exemples

### Exemple 1 - Composition selon l'invention

Un premier exemple de formulation comprenant une eau de pomme, et se présentant sous forme d'un gel douche est présenté dans le tableau 1, ci-après.

**[Tableau 1]**

| | **Ingrédients** | **%** |
|---|---|---|
| A | EAU DE FRUIT MALUS DOMESTICA | 95,635 |
| B | GOMME XANTHANE OUVRICARE D | 0,400 |
| C | PENTYLENE GLYCOL | 2,000 |
| | SODIUM BENZOATE | 0,300 |
| | SODIUM PHYTATE | 0,100 |
| D | TRIETHYL CITRATE | 1,000 |
| | PARFUM | 0,500 |
| | ACIDE CITRIQUE | 0,065 |
| | | 100,000 |

La composition de l'exemple 1 peut notamment être obtenue par le procédé suivant :
- Disperser B dans A sous agitation modérée.
- Ajouter progressivement sous agitation C dans AB.
- Ajouter progressivement sous agitation D dans ABC.
- Ajuster le pH à 5,0 - 5,5.
- Maintenir l'agitation pendant 15-20 minutes.

La composition se présente alors sous forme d'un gel douche avec une texture gélifiée et une viscosité comprise entre 55 et 80 mPa.s.

### Exemple 2 - Composition selon l'invention

Un second exemple de formulation comprenant une eau de raisin, et se présentant sous forme d'un gel douche est présenté dans le tableau 2, ci-après.

**[Tableau 2]**

| | **Ingrédients** | **%** |
|---|---|---|
| A | EAU DE FRUIT VITIS VINIFERA | 95,635 |
| B | GOMME XANTHANE OUVRICARE D | 0,400 |
| C | PENTYLENE GLYCOL | 2,000 |
| | SODIUM BENZOATE | 0,300 |
| | SODIUM PHYTATE | 0,100 |
| D | TRIETHYL CITRATE | 1,000 |
| | PARFUM | 0,500 |
| | ACIDE CITRIQUE | 0,065 |
| | | 100,000 |

La composition de l'exemple 2 peut notamment être obtenue par le procédé suivant :
- Disperser B dans A sous agitation modérée.
- Ajouter progressivement sous agitation C dans AB.
- Ajouter progressivement sous agitation D dans ABC.
- Ajuster le pH à 5,0 - 5,5.
- Maintenir l'agitation pendant 15-20 minutes.

La composition se présente alors sous forme d'un gel douche avec une texture gélifiée et une viscosité comprise entre 55 et 80 mPa.s.

### Exemple 3 - Composition hors invention

Un autre exemple de formulation comprenant une eau de pomme est présenté dans le tableau 3, ci-après.

**[Tableau 3]**

| | **Ingrédients** | **%** |
|---|---|---|
| A | EAU DE FRUIT MALUS DOMESTICA | 95,735 |
| B | GOMME XANTHANE OUVRICARE D | 0,300 |
| C | PENTYLENE GLYCOL | 2,000 |
| | SODIUM BENZOATE | 0,300 |
| | SODIUM PHYTATE | 0,100 |
| D | TRIETHYL CITRATE | 1,000 |
| | PARFUM | 0,500 |
| | ACIDE CITRIQUE | 0,065 |
| | | 100,000 |

La composition de l'exemple 3 peut notamment être obtenue par le procédé suivant :
- Disperser B dans A sous agitation modérée.
- Ajouter progressivement sous agitation C dans AB.
- Ajouter progressivement sous agitation D dans ABC.
- Ajuster le pH à 5,0 - 5,5.
- Maintenir l'agitation pendant 15-20 minutes.

La composition se présente alors sous forme d'un gel douche avec une texture liquide ne présentant aucune texture et aucune tenue. Le produit est alors trop liquide et ne répond pas à une appellation gel.

### Exemple 4 - Composition hors invention

Un autre exemple de formulation comprenant une eau de pomme et se présentant sous forme d'un gel douche est présenté dans le tableau 4, ci-après.

**[Tableau 4]**

| | **Ingrédients** | **%** |
|---|---|---|
| A | EAU DE FRUIT MALUS DOMESTICA | 95,535 |
| B | GOMME XANTHANE OUVRICARE D | 0,500 |
| C | PENTYLENE GLYCOL | 2,000 |
| | SODIUM BENZOATE | 0,300 |
| | SODIUM PHYTATE | 0,100 |
| D | TRIETHYL CITRATE | 1,000 |
| | PARFUM | 0,500 |
| | ACIDE CITRIQUE | 0,065 |
| | | 100,000 |

La composition de l'exemple 4 peut notamment être obtenue par le procédé suivant :
- Disperser B dans A sous agitation modérée.
- Ajouter progressivement sous agitation C dans AB.
- Ajouter progressivement sous agitation D dans ABC.
- Ajuster le pH à 5,0 - 5,5.
- Maintenir l'agitation pendant 15-20 minutes.

La composition se présente alors sous forme d'un gel douche avec une texture gel. Toutefois, le produit est alors trop épais et n'est pas satisfaisant en termes d'application, laissant également de nombreux résidus sur la peau et une sensation collante désagréable.

### Exemple 5 - Composition hors invention

Un autre exemple de formulation comprenant une eau de pomme, et se présentant sous forme d'un gel douche est présenté dans le tableau 5, ci-après.

**[Tableau 5]**

| | **Ingrédients** | **%** |
|---|---|---|
| A | EAU DE FRUIT MALUS DOMESTICA | 96,635 |
| B | GOMME XANTHANE OUVRICARE D | 0,400 |
| C | PENTYLENE GLYCOL | 0,600 |
| | SODIUM BENZOATE | 0,500 |
| | SODIUM PHYTATE | 0,300 |
| D | TRIETHYL CITRATE | 1,000 |
| | PARFUM | 0,500 |
| | ACIDE CITRIQUE | 0,065 |
| | | 100,000 |

La composition de l'exemple 5 peut notamment être obtenue par le procédé suivant :
- Disperser B dans A sous agitation modérée.
- Ajouter progressivement sous agitation C dans AB.
- Ajouter progressivement sous agitation D dans ABC.
- Ajuster le pH à 5,0 - 5,5.
- Maintenir l'agitation pendant 15-20 minutes.

La composition se présente alors sous forme d'un gel douche avec une texture gel. Toutefois, le produit est se dégrade rapidement. L'intégrité du produit dans le temps est alors compromise rendant difficile sa conservation dans le temps.

## Revendications

1. Composition cosmétique pour soins corporels sous forme d'une solution nettoyante gélifiée comprenant :
- au moins 90% d'eau, en poids du poids total de la composition,
- entre 1,5% et 4% d'au moins un conservateur, en poids du poids total de la composition,
- entre 0,35% et 0,45% d'au moins un agent gélifiant, en poids du poids total de la composition.

2. Composition selon la précédente revendication, **caractérisée en ce que** ladite composition est de type sans rinçage.

3. Composition selon l'une des précédentes revendications, **caractérisée en ce que** la composition présente une viscosité comprise entre 40 et 95 mPa.s, préférentiellement entre 55 et 80 mPa.s.

4. Composition selon l'une des précédentes revendications, **caractérisée en ce que** l'eau est une eau d'origine végétale.

5. Composition selon l'une des précédentes revendications, **caractérisée en ce que** l'eau d'origine végétale est choisie parmi le groupe constitué d'une eau de fruit, d'une eau de légume, d'une eau florale, d'une eau végétale d'une eau alimentaire et leur mélange.

6. Composition selon l'une des précédentes revendications, **caractérisée en ce que** ladite composition comprend un mélange de trois conservateurs, le sodium benzoate, le sodium phytate et le pentylène glycol.

7. Composition selon l'une des précédentes revendications, **caractérisée en ce que** l'agent gélifiant est la gomme de xanthane, préférentiellement la gomme de xanthane CAS N°11138-66-2.

8. Composition selon l'une des précédentes revendications, **caractérisée en ce que** ladite composition comprend 0,4% de gomme de xanthane CAS N°11138-66-2, en poids du poids total de la composition.

9. Composition selon l'une des précédentes revendications, **caractérisée en ce que** l'eau de fruit est une eau de pomme ou une eau de raisin.

10. Composition selon l'une des précédentes revendications, **caractérisée en ce que** ladite composition comprend :
- Entre 1,5% et 2,5% de pentylène glycol, en poids du poids total de la composition,
- Entre 0,2% et 0,4% de sodium benzoate, en poids du poids total de la composition,
- Au plus 0,25% de sodium phytate, en poids du poids total de la composition.

11. Composition selon l'une des précédentes revendications, **caractérisée en ce que** ladite composition comprend également au moins un agent antioxydant, et/ou au moins un agent solubilisant, et/ou au moins un parfum.

12. Utilisation cosmétique d'une composition selon l'une des précédentes revendications, pour éliminer les mauvaises odeurs, et/ou le sébum, et/ou la transpiration.

13. Méthode de nettoyage corporel sans consommation d'eau supplémentaire comprenant l'application d'une composition gélifiée selon l'une des revendications 1 à 10.

14. Méthode selon la revendication précédente, dans laquelle la méthode comprend une étape d'élimination sans rinçage et/ou sans essuyage.
